# EUROPEAN PATENT SPECIFICATION

(11) **EP 3 862 013 B1**
(45) Date of publication and mention of the grant of the patent: **07.08.2024**
(21) Application number: 19868817.8
(22) Date of filing: 04.10.2019
(51) Int. Cl.: A61K 38/10, A61K 35/28, A61K 35/38, A61K 45/00, A61P 1/04

(54) **PHARMACEUTICAL COMPOSITION FOR USE IN TREATING OR PREVENTING REFRACTORY ULCER IN THE INTESTINAL TRACT**
PHARMAZEUTISCHE ZUSAMMENSETZUNG ZUR VERWENDUNG IN DER BEHANDLUNG ODER VORBEUGUNG VON REFRAKTÄREN GESCHWÜREN IM DARMTRAKT
COMPOSITION PHARMACEUTIQUE POUR L'UTILISATION DANS LE TRAITEMENT OU LA PRÉVENTION D'UN ULCÈRE REFRACTAIRE DANS LE TRACTE INTESTINAL

(30) Priority: 05.10.2018 JP 2018189712
(43) Date of publication of application: 11.08.2021
(73) Proprietor: KURUME UNIVERSITY, Kurume-shi Fukuoka 830-0011 (JP); 3-D Matrix, Ltd., Chiyoda-ku Tokyo 102-0083 (JP)
(72) Inventor: YAMASAKI, Hiroshi, Kurume-shi, Fukuoka 830-0011 (JP); MITSUYAMA, Keichi, Kurume-shi, Fukuoka 830-0011 (JP); ARAKI, Toshihiro, Kurume-shi, Fukuoka 830-0011 (JP)
(74) Representative: EIP
(86) International application number: PCT/JP2019/039263
(87) International publication number: WO 2020/071527

(56) References cited:
- WO-A1-2014/141143
- WO-A1-2017/104723
- WO-A1-2017/158432
- WO-A1-2019/183625
- WO-A1-2019/200298
- WO-A2-2008/113030
- JP-A- 2016 515 113
- JP-A- 2017 159 044
- US-A1- 2008 032 934
- MATHIEU PIOCHE ET AL: "A self-assembling matrix-forming gel can be easily and safely applied to prevent delayed bleeding after endoscopic resections", ENDOSCOPY INTERNATIONAL OPEN, vol. 04, no. 04, 30 March 2016 (2016-03-30), DE, pages E415 - E419, XP055400864, ISSN: 2364-3722, DOI: 10.1055/s-0042-102879
- LEE M.F. ET AL: "A novel haemostatic agent based on self-assembling peptides in the setting of nasal endoscopic surgery, a case series", INTERNATIONAL JOURNAL OF SURGERY CASE REPORTS, vol. 41, 1 January 2017 (2017-01-01), AMSTERDAM, NL, pages 461 - 464, XP055922046, ISSN: 2210-2612, Retrieved from the Internet <URL:https://www.ncbi.nlm.nih.gov/pmc/articles/PMC5709301/pdf/main.pdf> DOI: 10.1016/j.ijscr.2017.11.024
- ARAKI T ET AL: "P1013 SELF-ASSEMBLING PEPTIDE HYDROGEL ENHANCES INTESTINAL BARRIER FUNCTION IN TOPICAL TNBS MODEL IN RATS", 27TH UEG WEEK 2019 BARCELONA, vol. 7, 1 October 2019 (2019-10-01), pages 590 - 590, XP055921849, Retrieved from the Internet <URL:https://onlinelibrary.wiley.com/doi/epdf/10.1177/2050640619854671>
- BURY MATTHEW I ET AL: "Anti-Inflammatory Supramolecular Nanofibers to Modulate Inflammation in a Mouse Model of Ileitis", GASTROENTEROLOGY, ELSEVIER INC, US, 22 April 2017 (2017-04-22), XP085103923, ISSN: 0016-5085, DOI: 10.1016/S0016-5085(17)32061-9
- URAOKA, T. ET AL.: "A novel fully synthetic and self-assembled peptide solution for endoscopic submucosal dissection-induced ulcer in the stomach", GASTROINTESTINAL ENDOSCOPY, vol. 83, no. 6, 2016, pages 1259 - 1264, XP029537832, DOI: 10.1016/j.gie.2015.11.015
- PIOCHE, M. ET AL.: "A self-assembling matrix-forming gel can be easily and safely applied to prevent delayed bleeding after endoscopic resections", ENDOSCOPY INTERNATIONAL OPEN, April 2016 (2016-04-01), pages E415 - E419, XP055400864, DOI: 10.1055/s-0042-102879

## Description

### Technical Field

The present invention relates to a pharmaceutical composition comprises a self-assembling peptide containing RADARADARADARADA (SEQ ID NO: 1), for use in a method of treating or preventing a refractory ulcer in the intestinal tract, wherein the ulcer in the intestinal tract is caused by ulcerative colitis, Crohn' s disease or Behcet's disease.

### Background Art

The intestine is an organ that digests and absorbs nutrients and water necessary for the vital activities of living organisms. On the other hand, the intestine also has an immune defense function for eliminating foreign substances such as pathogens. The intestine is responsible for maintaining life by controlling these conflicting properties in a well-balanced manner. However, it is known that when these functional balances are abnormal, this equilibrium state is disrupted and various intestinal diseases are caused.

In particular, inflammatory bowel disease (inflammatory bowel disease; IBD), of which the number of patients has been increasing in recent years, is a general term for chronic diseases that give rise to inflammation of unknown cause of the intestinal tract; it is an intractable disease of unknown cause leading to long-term diarrhea and bloody stools and is classified as ulcerative colitis (ulcerative colitis; UC) and Crohn's disease (Crohn's disease; CD). Nutrition therapy, drug therapy, surgical therapy, endoscopic treatment, and the like are mainly employed as treatment methods for inflammatory bowel disease. However, in many cases, inflammatory bowel disease symptoms repeatedly abate and worsen over a long period of time.

Inflammatory bowel disease often forms refractory ulcers, and as the pathological condition progresses, fistulas may also form. However, an extremely effective therapeutic drug and treatment method for improving these pathological conditions have not been established to date.

Self-assembling peptides have a property of forming a self-assembled structure in which a large number of peptide molecules are regularly arranged according to their amino acid sequence. In recent years, due to their physical, chemical, and biological properties, such peptides have attracted attention as materials that can be used for various medical applications (for example, Patent Literature 1 and Patent Literature 2).

WO2014141143 discloses that human patients with lesions in the esophagus, stomach or colorectum underwent esophageal, gastric, and colorectal endoscopic submucosal dissection (ESD). After the ESD procedure, the self-assembling peptide RADA16 was administered to a target area in the subject. Bleeding was stopped.

### Prior Art Literature

### Patent Literature

[Patent Literature 1] WO 2013/133413
[Patent Literature 2] WO 2015/194194

### Summary of Invention

### Problem to Be Solved by Invention

An object of the present invention is to provide an effective treatment for a refractory ulcer in the intestinal tract, wherein the ulcer in the intestinal tract is caused by ulcerative colitis, Crohn' s disease or Behcet's disease.

### Means for Solving Problem

When the inventors of the present invention diligently searched for an effective treatment a refractory ulcer in the intestinal tract, wherein the ulcer in the intestinal tract is caused by ulcerative colitis, Crohn' s disease or Behcet's disease, surprisingly, a composition containing a self-assembling peptide was found to be effective in in the treatment, which led to the completion of the present invention.

That is, in one embodiment, the present invention relates to a pharmaceutical composition comprising a self-assembling peptide containing RADARADARADARADA (SEQ ID NO: 1), for use in a method of treating or preventing a refractory ulcer in the intestinal tract, wherein the ulcer in the intestinal tract is caused by ulcerative colitis, Crohn' s disease or Behcet's disease.

In a preferred embodiment of the present invention, the pharmaceutical composition is applied to the ulcer of a subject having an ulcer in the intestinal tract.

In a preferred embodiment of the present invention, the pharmaceutical composition is locally applied to an ulcer site of a subject using an endoscope or anoscope.

In a preferred embodiment of the invention, the pharmaceutical composition contains the self-assembling peptide at a concentration of 0.1% to 10.0% by weight.

In a preferred embodiment of the present invention, the pharmaceutical composition further contains an additional drug effective in treating or preventing an ulcer in the intestinal tract, or is used in combination with an additional drug effective in treating or preventing an ulcer in the intestinal tract

In a preferred embodiment of the present invention, the additional drug effective in treating or preventing the ulcer in the intestinal tract is characterized by one or two or more drugs selected from the group consisting of an antidiarrheal agent, an anti-inflammatory agent, a corticosteroid, an immunomodulatory agent or immunosuppressive agent, an intestinal mucosal protective agent, and a blood flow accelerating agent.

In a preferred embodiment of the present invention, the additional drug effective in treating or preventing the ulcer in the intestinal tract is characterized by a composition containing a cell sheet, an intestinal stem cell, a hematopoietic stem cell, an adipose stem cell, or a mesenchymal stem cell.

In a preferred embodiment of the present invention, the self-assembling peptide is characterized in that it undergoes gelation by self-assembling when the composition is applied to an ulcer site site of a subject.

In a preferred embodiment of the present invention, the self-assembling peptide is characterized by a peptide consisting of RADARADARADARADA (SEQ ID NO: 1).

### Brief Description of Drawings

[FIG. 1] FIG. 1 illustrates a procedure for producing a Trinitrobenzene sulphonic acid (TNBS)-induced colonic ulcer model rat and an experimental procedure of an example.
[FIG. 2] FIG. 2 illustrates a specific procedure for producing a TNBS-induced colonic ulcer model rat and an experimental procedure of an example.
[FIG. 3] FIG. 3 is a photograph taken during endoscopic administration of a self-assembling peptide solution used in the present invention to an ulcer site on day 2 and day 4.
[FIG. 4] FIG. 4 illustrates changes in body weight of rats in a PuraMatrix application group and a control group from day 1 to day 7. In the drawing, "Cont" indicates the control group (Control), and "PM" indicates the PuraMatrix administration group.
[FIG. 5] FIG. 5 illustrates changes over time of the ulcer site in the PuraMatrix application group and the control group.
[FIG. 6] FIG. 6 illustrates the ulcer area and intestinal weight in the PuraMatrix application group and the control group.
[FIG. 7] FIG. 7 illustrates the expression levels of IL-1α, IL-1β, IL-6, and TNF-α in tissues of the PuraMatrix application group and the control group.
[FIG. 8] FIG. 8 illustrates the expression levels of TTF-1, Foxe1 and ZO-1 in tissues of the PuraMatrix application group and the control group.
[FIG. 9] FIG. 9 illustrates the expression levels of Claudin 1, Claudin 2, Claudin 3, and Occludin in tissues of the PuraMatrix application group and the control group.
[FIG. 10] FIG. 10 illustrates the expression levels of VEGFA and HGF in tissues of the PuraMatrix application group and the control group.

### Description of Embodiments

The present invention relates to a pharmaceutical composition comprising a self-assembling peptide containing RADARADARADARADA (SEQ ID NO: 1), for use in a method of treating or preventing a refractory ulcer in the intestinal tract, wherein the ulcer in the intestinal tract is caused by ulcerative colitis, Crohn' s disease or Behcet's disease. The present invention has been developed specifically for treating refractory ulcers caused by inflammatory bowel disease (ulcerative colitis and Crohn's disease), yet such refractory ulcers in the intestinal tract caused by Behcet's disease can also be treated.

Here, in the present specification, a "refractory" ulcer or fistula may mean an ulcer or a fistula exhibiting resistance to, for example, a general conservative treatment (for example, nutrition therapy, drug therapy, or the like) for inflammatory bowel disease.

The application subject of the present invention may be human or non-human. The non-human subject may be, for example, a non-human animal, such as a non-human mammal, a bird, a reptile, an amphibian, or a fish. Examples of the non-human mammal may include rodents (for example, mice and rats), dogs, cats, horses, pigs, cows, sheep, goats, primates, and the like.

The administration method and route of administration of the pharmaceutical composition for use according to the present invention are not limited, but it is particularly desirable that the administration method and the route of administration allow local administration to a lesion site. For example, topical administration using an endoscope or anoscope, administration using a nasogastrointestinal tube or gastrointestinal fistula tube, administration via a surgical incision site of the intestinal tract, and the like may be employed.

The dose and frequency of administration of the pharmaceutical composition of the present invention may be appropriately determined by a person having ordinary skill in the art (for example, a doctor) according to the pathological condition of the subject.

The concentration of the self-assembling peptide in the pharmaceutical composition of the present invention may be 0.1% by weight to 10.0% by weight, preferably 0.3% by weight to 8.0% by weight, more preferably, 0.5% by weight to 5.0% by weight, and most preferably 1.0% by weight to 3.0% by weight.

The pharmaceutical composition for use according to the present invention may further contain an additional drug effective in treating or preventing an ulcer in the intestinal tract, or may be used in combination with an additional drug effective in treating or preventing an ulcer in the intestinal tract. An aspect of concomitant use of the pharmaceutical composition for use according to the present invention and an additional drug is not limited, and for example, it may be an aspect wherein drugs each prepared separately are administered to a subject at the same time, or an aspect wherein each drug is administered to a subject at different times.

An additional drug that may be used together with the pharmaceutical composition for use according to the present invention may be, for example, a drug commonly used for treating inflammatory bowel disease (ulcerative colitis and Crohn's disease). Examples of drugs commonly used to treat inflammatory bowel disease (ulcerative colitis and Crohn's disease) may include antidiarrheal agents, anti-inflammatory agents, corticosteroids, immunomodulatory agents or immunosuppressive agents, intestinal mucosal protective agents, and blood flow accelerating agents.

Examples of an antidiarrheal agent that may be used together with the pharmaceutical composition for use according to the present invention include diphenoxylate, loperamide, deodorized opium tincture, and codeine. Examples of an anti-inflammatory agent that may be used together with the pharmaceutical composition of the present invention include salazosulfapyridine and related drugs thereof (mesalazine, olsalazine, balsalazide, and the like). Examples of a corticosteroid that may be used together with the pharmaceutical composition of the present invention include prednisolone, budesonide, and hydrocortisone. Examples of an immunomodulatory agent or immunosuppressive agent that may be used together with the pharmaceutical composition for use according to the present invention include tacrolimus, azathioprine, mercaptopurine, cyclosporine, infliximab, adalibumab, and golimumab.

An additional drug that may be used together with the pharmaceutical compositions for use according to the present invention may be, for example, a composition containing a cell sheet, an intestinal stem cell, a hematopoietic stem cell, an adipose stem cell, or a mesenchymal stem cell. Development of a mucosal regeneration treatment method for inflammatory bowel disease patients utilizing cell sheets, stem cells, and the like is underway, and a person having ordinary skill in the art may employ concomitant use of a treatment using the pharmaceutical composition for use according to the present invention and a mucosal regeneration treatment method utilizing cell sheets, stem cells, and the like. Furthermore, a synergistic effect may be expected by mixing a cell sheet, stem cells, and the like with the pharmaceutical composition of the present invention and applying the mixture to a subject.

The pharmaceutical composition for use according to the present invention contains a self-assembling peptide. In the present specification, peptides "self-assembling" in solution means that peptides spontaneously assemble in solution via some sort of interaction (for example, electrostatic interaction, hydrogen bond, van der Waals forces, hydrophobic interaction, and the like) and this should not be interpreted in a limited sense. In the present invention, a self-assembling peptide refers to a peptide having a property of forming a self-assembled structure in which a large number of peptide molecules are regularly arranged according to their amino acid sequence. Also, due to its nature, when a composition containing self-assembling peptides is applied to a disease site of a subject, the self-assembling peptides self-assemble to form a gel at the applied site.

Note, regarding the self-assembling peptide used in the present invention, an aqueous solution (that is, a peptide aqueous solution prior to the self-assembling peptides self-assembling) itself may have a fixed viscosity prior to application to the subject. However, in the present specification, for the convenience of description, even when the peptide aqueous solution prior to application to the subject has a fixed viscosity, it may be referred to as a "peptide solution (or peptide aqueous solution)". Furthermore, even when the peptide aqueous solution prior to application to the subject has a fixed viscosity, self-assembly of the self-assembling peptide occurs after application of the aqueous solution to the subject, and the viscosity of the composition further increases, which is sometimes referred to as "gelation (or gel formation)".

The self-assembling peptide used in the present invention contains a peptide structure such as the foregoing, so that when a β-sheet structure is formed in an aqueous solution, only polar amino acid residues may be disposed on one surface of the β-sheet structure, and only non-polar amino acid residues may be disposed on the other surface. Accordingly, such a β-sheet structure may form a two-structure layer, assembled so as to hide the hydrophobic surface (the surface on which only non-polar amino acid residues are disposed). Then, as self-assembly of the molecule progresses, the layer structure of this β-sheet elongates, allowing a three-dimensional spatial structure (for example, hydrogel) to be formed. Note that the "self-assembling peptide" used in the present invention may sometimes be referred to as a "self-assembled peptide".

In the present invention, "amino acid" is used in the broadest sense, and includes not only proteinogenic amino acids but also non-proteinogenic amino acids such as amino acid variants and derivatives. Considering this broad definition, a person having ordinary skill in the art would likely understand that examples of the amino acid in the present invention include proteinogenic L-amino acids; D-amino acids; chemically modified amino acids, such as amino acid variants and derivatives; non-proteinogenic amino acids, such as norleucine, β-alanine, and ornithine; chemically synthesized compounds having properties known in the field as being characteristic of amino acids; and the like. Examples of non-proteinogenic amino acids include α-methyl amino acids (α-methylalanine and the like), D-amino acids, histidine-like amino acids (2-amino-histidine, β-hydroxy-histidine, homohistidine, α-fluoromethyl-histidine, α-methyl-histidine, and the like), amino acids having extra methylene in the side chain ("homo" amino acids), and amino acids in which the carboxylic acid functional group amino acid in the side chain is substituted with a sulfonic acid group (cysteic acid and the like). In a preferred aspect of the present invention, the amino acid used in the present invention may be a proteinogenic amino acid.

Note that in the specification, a notation such as, for example, "aspartic acid (Asp: D)" means the three-letter notation "Asp" and the one-letter notation "D" are sometimes used as abbreviations of aspartic acid.

The self-assembling peptide containing RADARADARADARADA (SEQ ID NO: 1) may be a repeating sequence of "RADA" (repeating up to 8 times, and preferably repeating up to 6 times), more preferably a peptide containing RADARADARADARADA (SEQ ID NO: 1). More preferably, the self-assembling peptide used in the present invention may be a peptide consisting of RADARADARADARADA (SEQ ID NO: 1).

The self-assembling peptide used in the present invention may be modified (or labeled) as long as they do not lose the main properties of the peptides intended by the present invention, and such modified (or labeled) peptides are also included in the "self-assembling peptide" in the present invention. The method for modifying (or labeling) the self-assembling peptide used in the present invention may be arbitrarily selected by a person having ordinary skill in the art, and examples may be an addition of a functional group or the like, addition of a chemical substance, or addition of a further protein or peptide. Examples of an addition of a functional group or the like include acylation, acetylation, alkylation, amidation, biotinylation, formylation, carboxylation, glutamylation, glycosylation (addition of sugar chains), glycylation, hydroxylation, isoprenylation, lipoylation, addition of a nucleotide or derivative thereof, polyethylene glycol (PEG) formation, and addition of lipid chains. Furthermore, an example of an addition of a chemical substance includes a suitable labeling agent, for example, an addition such as a radioisotope (examples: ¹²⁵ I, ¹³¹ I, ³ H, ¹⁴ C, and the like), an enzyme (examples: β-galactosidase, β-glucosidase, alkaline phosphatase, peroxidases, malate dehydrogenase, and the like), a fluorescent substance (examples: luminol, luminol derivatives, luciferin, lucigenin, and the like), an affinity tag (example: biotin or the like), or the like. Furthermore, examples of additions of further proteins or peptides include ISGylation, SUMOylation, and ubiquitination.

Note that the terms used in the present specification are used to describe a defined embodiment and are not intended to limit the invention.

Furthermore, the term "containing" as used in the present specification is intended to include the existence of described matters (members, steps, elements, numbers, and the like) unless a different understanding is clearly required in light of the context, and it does not eliminate the existence of other matters (members, steps, elements, numbers, and the like).

All terms used herein (including technical terms and scientific terms ) have the same meanings as widely understood by a person having ordinary skill in the art to which the present invention belongs, unless there is a different meaning. The terms used herein should be interpreted as having meanings consistent with the meanings in the present specification and related fields, unless a different definition is clearly stated, and they should not be interpreted in an idealized or overly formal sense.

Occasionally terms such as first, second, and the like are used to present various elements, but it is understood that these elements should not be limited by those terms. These terms are used only to distinguish one element from another; for example, referring to a first element as a second element, and similarly noting the second element as the first element is possible without departing from the scope of the present invention.

Hereinafter, the present invention will be described in more detail with reference to examples; however, the present invention may be embodied in various forms and should not be interpreted as being limited to the examples described herein.

### Examples

### [Example 1]

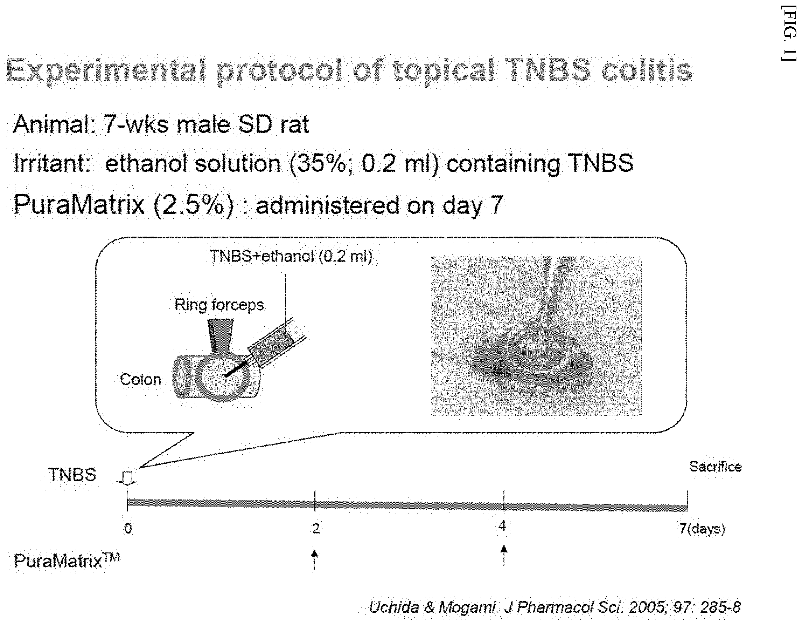

A trinitrobenzene sulphonic acid (TNBS)-induced colonic ulcer model rat was prepared based on a method (FIG. 1) described in Uchida. J Pharmacol Sci 2005, and an ulcer repairing effect of the pharmaceutical composition for use according to the present invention was examined.

### Method

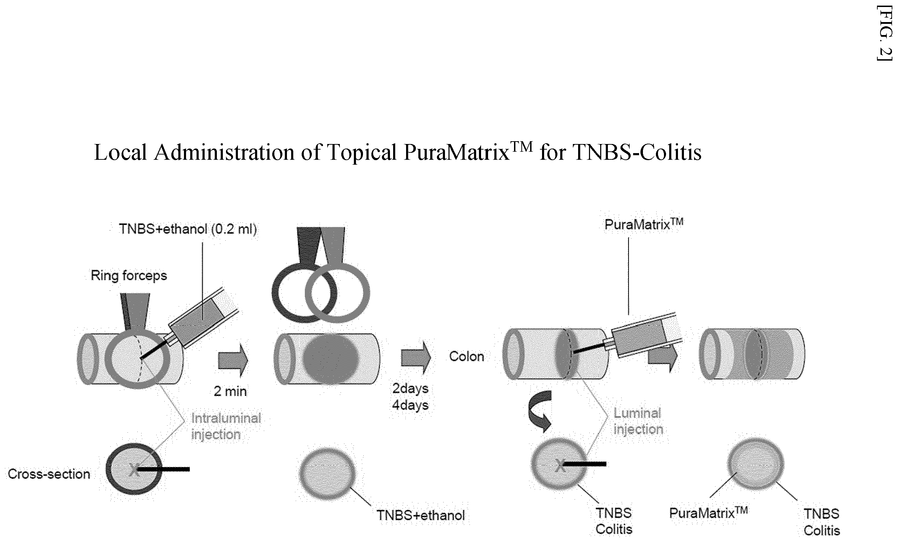

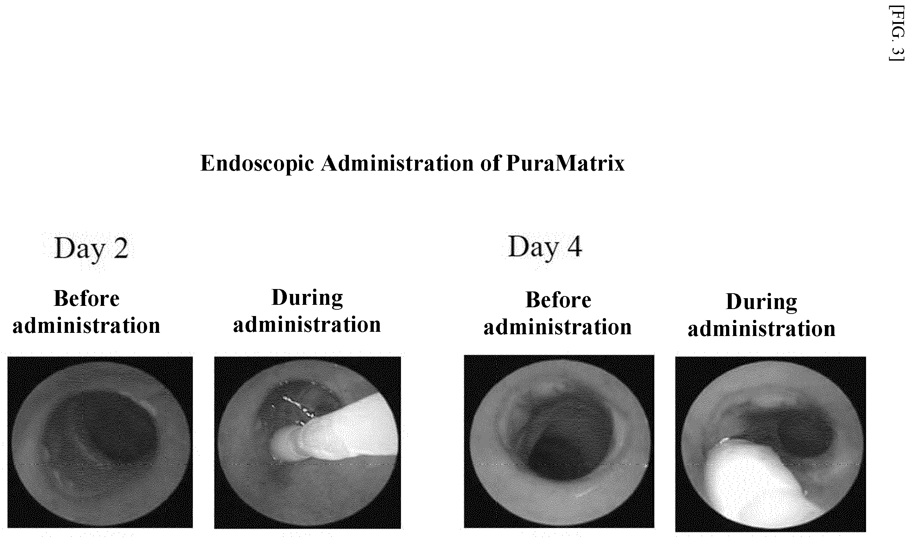

Following laparotomy of a 7-week-old male SD rat under anesthesia, a portion of the colon was fixed using ring tweezers, and 0.2 mL of a TNBS and ethano1 solution was injected into the lumen to prepare a localized ulcer model. On day 2, a self-assembling peptide solution for use according to the present invention (PuraMatrix (trademark), 3-D Matrix, Ltd., Tokyo) (n = 6) or physiological saline (n = 7) was applied to the ulcer portion under colonoscopy observation, and the ulcer size was observed using an endoscope on day 2, day 4, and day 7. On day 7, the rat was sacrificed and the ulcer area of the colon, intestinal weight (g/3 cm), and histological findings were evaluated (FIG. 2 and FIG. 3).

The "PuraMatrix" used in the present example is a composition containing the self-assembling peptide RADARADARADARADA (SEQ ID NO: 1) at a concentration of 2.5% by weight.

### Result

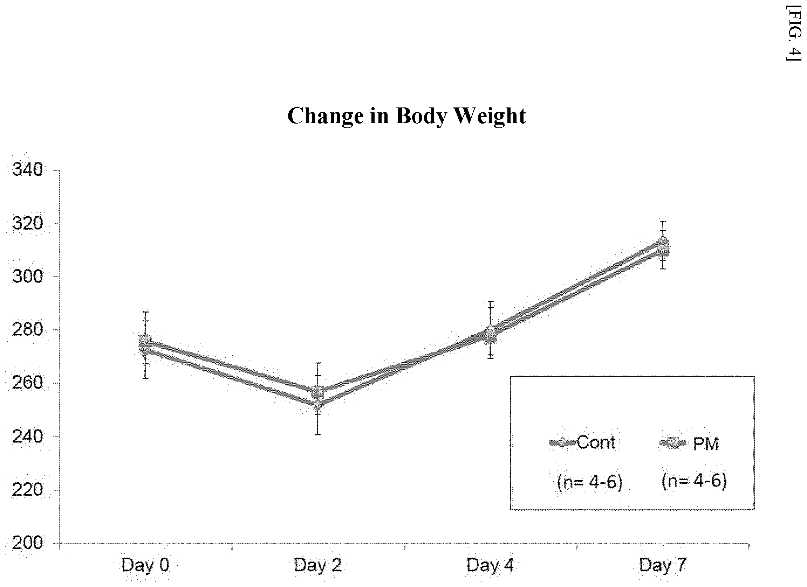

Changes in rat body weight were similar in the PuraMatrix application group and the control group (FIG. 4).

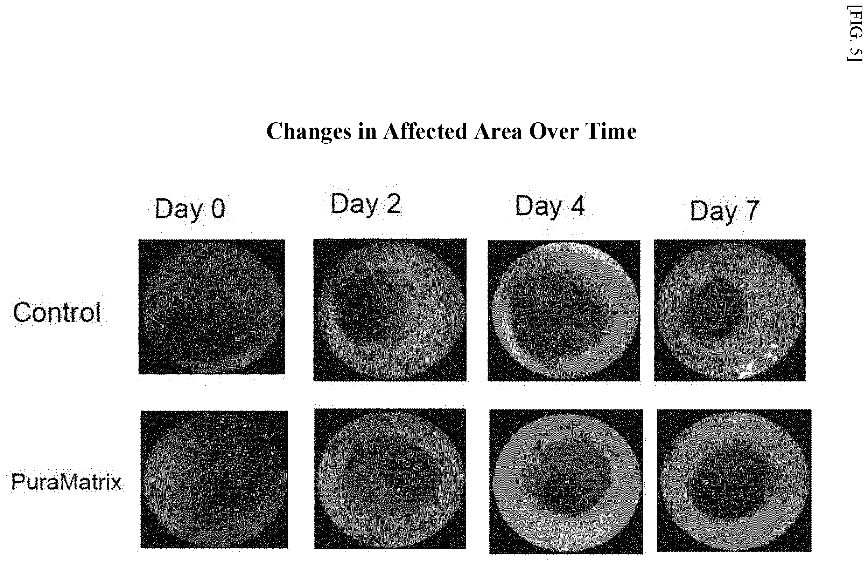

Endoscopic evaluation: In the PuraMatrix application group, the ulcer size reduced as compared to the control group (FIG. 5).

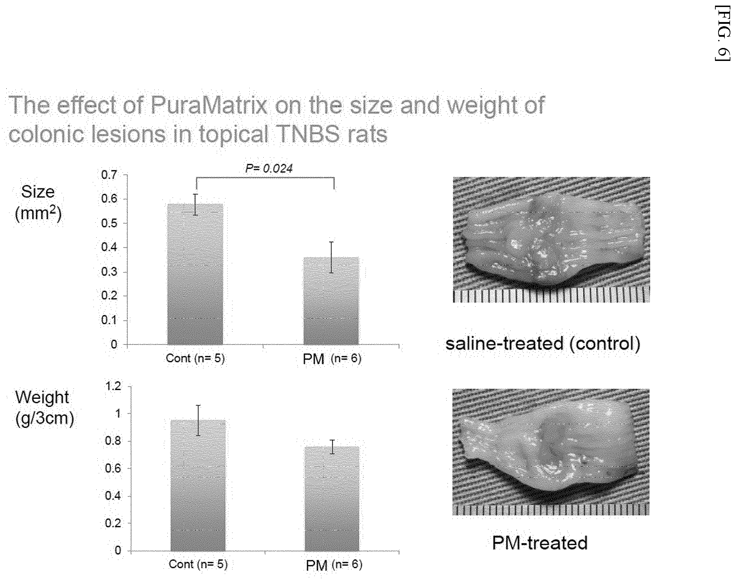

Post-sacrifice evaluation: The ulcer area reduced significantly in the PuraMatrix application group as compared to the control group (P = 0.024). The intestinal weight also tended to decrease in the PuraMatrix application group as compared to the control group (FIG. 6).

### Conclusion

From the above results, it has been shown that the pharmaceutical composition for use according to the present invention is effective in treating refractory ulcers in the intestinal tract in inflammatory bowel disease.

### [Example 2]

A colonic ulcer model rat was prepared according to the same procedure as in Example 1, and the ulcer repairing effect of the pharmaceutical composition for use according to the present invention was examined from the molecular level.

### Method

A colonic ulcer model rat was prepared according to the same procedure as in Example 1, and the self-assembling peptide solution for use according to the present invention (PuraMatrix (PM): n = 6) or physiological saline (Control: n = 7) was administered to the ulcer site, and on day 7 tissue at the ulcer site was collected. The amount of various cytokines in the collected tissues was measured by PCR.

### Result

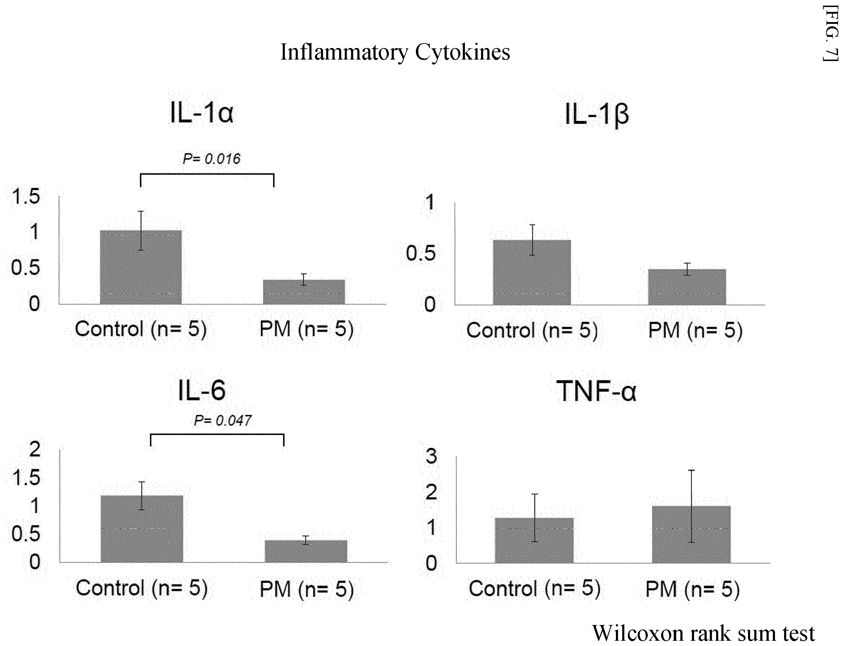

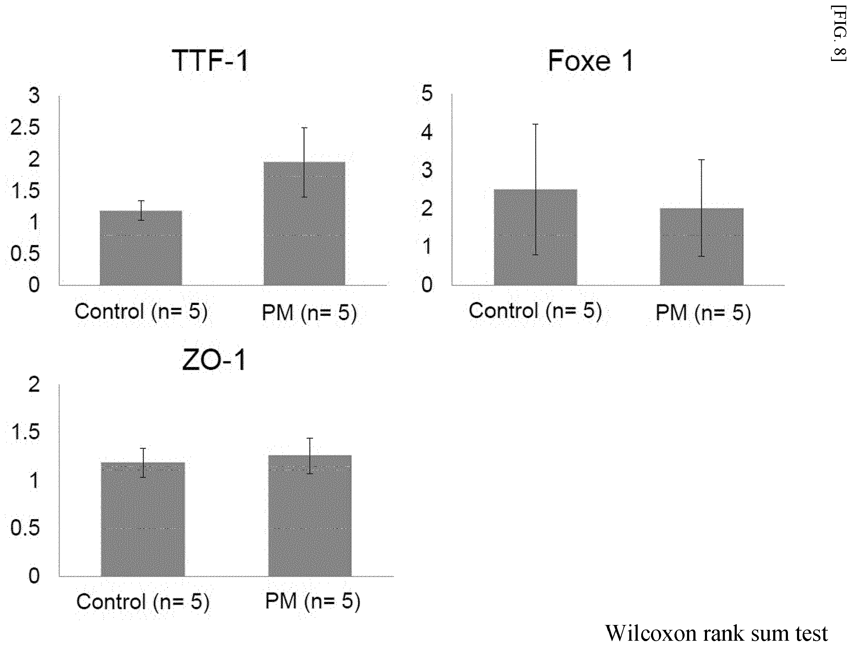

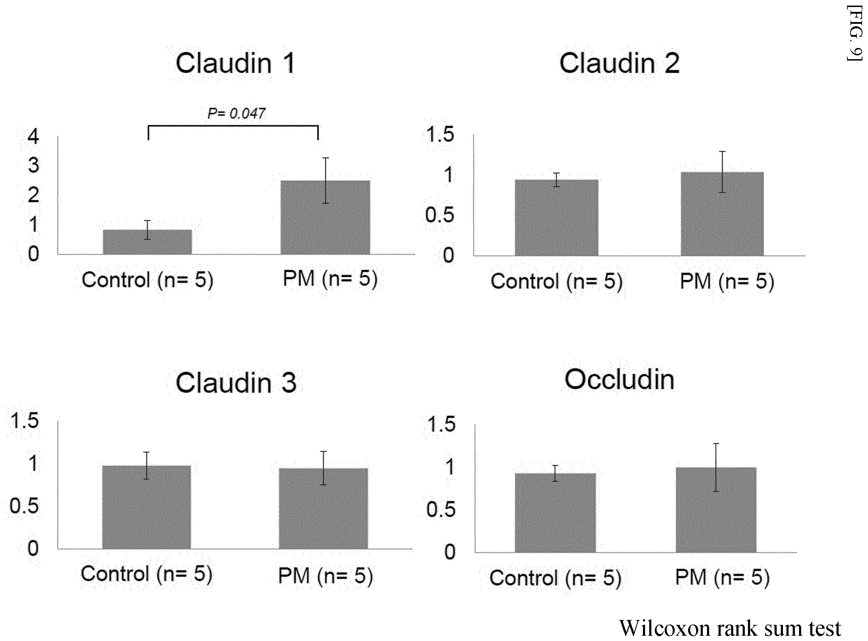

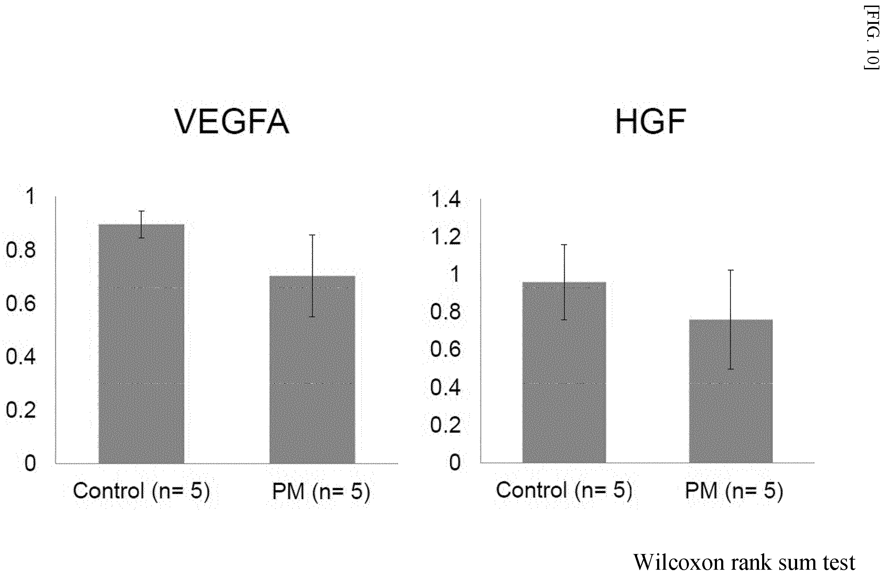

The results are shown in FIGS. 7 to 10. Among the various cytokines measured, expression of inflammatory cytokines IL-1a and IL-6 was significantly reduced in the group to which the pharmaceutical composition for use according to the present invention was administered (FIG. 7).
Furthermore, regarding Claudin 1, which is a membrane protein having an intestinal protective effect, a significant increase in expression was observed in the group to which the pharmaceutical composition for use according to the present invention was administered (FIG. 9).

### Conclusion

From the above results, it has been shown that even at the molecular level, the pharmaceutical composition for use according to the present invention is effective in treating refractory ulcers in the intestinal tract in inflammatory bowel disease.

## Claims

1. A pharmaceutical composition comprising a therapeutically effective amount of a self-assembling peptide containing RADARADARADARADA (SEQ ID NO: 1), for use in a method of treating or preventing a refractory ulcer in the intestinal tract, wherein the ulcer in the intestinal tract is caused by ulcerative colitis, Crohn' s disease or Behcet's disease.

2. The pharmaceutical composition for use according to claim 1, wherein the pharmaceutical composition is applied to the ulcer of a subject having an ulcer in the intestinal tract.

3. The pharmaceutical composition for use according to claim 2, wherein the pharmaceutical composition is locally applied to the ulcer using an endoscope or an anoscope

4. The pharmaceutical composition for use according to any one of the preceding claims, wherein the pharmaceutical composition contains the self-assembling peptide at a concentration of 0.1% by weight to 10.0% by weight.

5. The pharmaceutical composition for use according to any one of the preceding claims, wherein the pharmaceutical composition further contains an additional drug effective in treating or preventing an ulcer in the intestinal tract.

6. The pharmaceutical composition for use according to any one of claims 1-4, wherein the pharmaceutical composition is used in combination with an additional drug effective in treating or preventing an ulcer in the intestinal tract.

7. The pharmaceutical composition for use according to claims 5 or 6, wherein the additional drug is one or two or more drugs selected from: an antidiarrheal agent, an anti-inflammatory agent, a corticosteroid, an immunomodulatory agent or immunosuppressive agent, an intestinal mucosal protective agent, and a blood flow accelerating agent.

8. The pharmaceutical composition for use according to claims 5 or 6, wherein the additional drug is a composition containing a cell sheet, an intestinal stem cell, a hematopoietic stem cell, an adipose stem cell, or a mesenchymal stem cell.

9. The pharmaceutical composition for use according to any one of the preceding claims,
wherein the self-assembling peptide undergoes gelation by self-assembling when the composition is applied to an ulcer of a subject.

10. The pharmaceutical composition for use according to any one of the preceding claims, wherein the self-assembling peptide is a peptide consisting of RADARADARADARADA (SEQ ID NO: 1).

## Patentansprüche

1. Pharmazeutische Zusammensetzung, umfassend eine therapeutisch wirksame Menge eines selbstassemblierenden Peptids, das RADARADARADARADA (SEQ ID NO: 1) enthält, zur Verwendung in einem Verfahren zum Behandeln oder Vorbeugung eines refraktären Geschwürs in dem Darmtrakt, wobei das Geschwür in dem Darmtrakt durch Colitis ulcerosa, Morbus Crohn oder Morbus Behcet verursacht wird.

2. Pharmazeutische Zusammensetzung zur Verwendung nach Anspruch 1, wobei die pharmazeutische Zusammensetzung auf das Geschwür einer Person aufgetragen wird, die ein Geschwür in dem Darmtrakt aufweist.

3. Pharmazeutische Zusammensetzung zur Verwendung nach Anspruch 2, wobei die pharmazeutische Zusammensetzung lokal auf das Geschwür unter Verwendung eines Endoskops oder eines Anoskops aufgetragen wird.

4. Pharmazeutische Zusammensetzung zur Verwendung nach einem der vorstehenden Ansprüche, wobei die pharmazeutische Zusammensetzung das selbstassemblierende Peptid in einer Konzentration von 0,1 Gew.-% bis 10,0 Gew.-% enthält.

5. Pharmazeutische Zusammensetzung zur Verwendung nach einem der vorstehenden Ansprüche, wobei die pharmazeutische Zusammensetzung ferner ein zusätzliches Arzneimittel enthält, das bei der Behandlung oder Vorbeugung eines Geschwürs in dem Darmtrakt wirksam ist.

6. Pharmazeutische Zusammensetzung zur Verwendung nach einem der Ansprüche 1 bis 4, wobei die pharmazeutische Zusammensetzung in Kombination mit einem zusätzlichen Arzneimittel verwendet wird, das bei der Behandlung oder Vorbeugung eines Geschwürs in dem Darmtrakt wirksam ist.

7. Pharmazeutische Zusammensetzung zur Verwendung nach den Ansprüchen 5 oder 6, wobei es sich bei dem zusätzlichen Arzneimittel um ein oder zwei oder mehr Arzneimittel handelt, ausgewählt aus: einem Mittel gegen Durchfall, einem entzündungshemmenden Mittel, einem Kortikosteroid, einem immunmodulierenden Mittel oder einem immunsupprimierenden Mittel, einem Mittel zum Schutz der Darmschleimhaut und einem Mittel zur Beschleunigung des Blutflusses.

8. Pharmazeutische Zusammensetzung zur Verwendung nach den Ansprüchen 5 oder 6, wobei das zusätzliche Arzneimittel eine Zusammensetzung ist, die eine Zellschicht, eine Darmstammzelle, eine hämatopoetische Stammzelle, eine Fettstammzelle oder eine mesenchymale Stammzelle enthält.

9. Pharmazeutische Zusammensetzung zur Verwendung nach einem der vorstehenden Ansprüche, wobei das selbstassemblierende Peptid eine Gelierung durch Selbstassemblierung erfährt, wenn die Zusammensetzung auf ein Geschwür einer Person aufgetragen wird.

10. Pharmazeutische Zusammensetzung zur Verwendung nach einem der vorstehenden Ansprüche, wobei das selbstassemblierende Peptid ein Peptid ist, das aus RADARADARADARADA (SEQ ID NO: 1) besteht.

## Revendications

1. Composition pharmaceutique comprenant une quantité thérapeutiquement efficace d'un peptide auto-assemblé contenant RADARADARADARADA (SEQ ID NO : 1), pour utilisation dans un procédé de traitement ou de prévention d'un ulcère réfractaire dans le tractus intestinal, dans laquelle l'ulcère dans le tractus intestinal est causé par la colite ulcéreuse, la maladie de Crohn ou la maladie de Behcet.

2. Composition pharmaceutique pour utilisation selon la revendication 1, dans laquelle la composition pharmaceutique est appliquée sur l'ulcère d'un sujet souffrant d'un ulcère dans le tractus intestinal.

3. Composition pharmaceutique pour utilisation selon la revendication 2, dans laquelle la composition pharmaceutique est appliquée localement sur l'ulcère à l'aide d'un endoscope ou d'un anoscope.

4. Composition pharmaceutique pour utilisation selon l'une quelconque des revendications précédentes, dans laquelle la composition pharmaceutique contient le peptide auto-assemblé à une concentration de 0,1 % en poids à 10,0 % en poids.

5. Composition pharmaceutique pour utilisation selon l'une quelconque des revendications précédentes, dans laquelle la composition pharmaceutique contient en outre un médicament supplémentaire efficace pour traiter ou prévenir un ulcère dans le tractus intestinal.

6. Composition pharmaceutique pour utilisation selon l'une quelconque des revendications 1 à 4, dans laquelle la composition pharmaceutique est utilisée en association avec un autre médicament efficace pour traiter ou prévenir un ulcère dans le tractus intestinal.

7. Composition pharmaceutique pour utilisation selon l'une quelconque revendications 5 ou 6, dans laquelle le médicament supplémentaire est un ou deux médicaments ou plus choisis parmi : un agent antidiarrhéique, un agent antiinflammatoire, un corticostéroïde, un agent immunomodulateur ou un agent immunosuppresseur, un agent protecteur de la muqueuse intestinale et un agent accélérateur de la circulation sanguine.

8. Composition pharmaceutique pour utilisation selon les revendications 5 ou 6, dans laquelle le médicament supplémentaire est une composition contenant un feuillet cellulaire, une cellule souche intestinale, une cellule souche hématopoïétique, une cellule souche adipeuse ou une cellule souche mésenchymateuse.

9. Composition pharmaceutique pour utilisation selon l'une quelconque des revendications précédentes, dans laquelle le peptide auto-assemblé subit une gélification par auto-assemblage lorsque la composition est appliquée sur un ulcère d'un sujet.

10. Composition pharmaceutique pour utilisation selon l'une quelconque des revendications précédentes, dans laquelle le peptide auto-assemblé est un peptide constitué de RADARADARADARADA (SEQ ID NO : 1).
